(19) Europäisches Patentamt
European Patent Office
Office européen des brevets

(11) EP 0 811 003 B1

(12) **EUROPÄISCHE PATENTSCHRIFT**

(45) Veröffentlichungstag und Bekanntmachung des Hinweises auf die Patenterteilung:
**02.09.1998 Patentblatt 1998/36**

(21) Anmeldenummer: **96904060.9**

(22) Anmeldetag: **10.02.1996**

(51) Int Cl.[6]: **C07D 301/14**

(86) Internationale Anmeldenummer:
**PCT/EP96/00578**

(87) Internationale Veröffentlichungsnummer:
**WO 96/26198 (29.08.1996 Gazette 1996/39)**

(54) **VERFAHREN ZUR HERSTELLUNG VON EPOXIDEN MITTELS AROMATISCHER PEROXYCARBONSÄUREN**

PROCESS FOR PRODUCING EPOXIDES USING AROMATIC PEROXYCARBOXYLIC ACIDS

PROCEDE DE PRODUCTION D'EPOXYDES AU MOYEN D'ACIDES PEROXYCARBOXYLIQUES AROMATIQUES

(84) Benannte Vertragsstaaten:
**BE CH DE ES FR GB IT LI NL**

(30) Priorität: **21.02.1995 DE 19505858**

(43) Veröffentlichungstag der Anmeldung:
**10.12.1997 Patentblatt 1997/50**

(73) Patentinhaber: **BASF AKTIENGESELLSCHAFT
67056 Ludwigshafen (DE)**

(72) Erfinder:
- **TELES, Joaquim, Henrique
D-67059 Ludwigshafen (DE)**
- **SCHNURR, Werner
D-67273 Herxheim (DE)**
- **FISCHER, Rolf
D-69121 Heidelberg (DE)**
- **RIEBER, Norbert
D-68259 Mannheim (DE)**
- **SCHULZ, Michael
D-67067 Ludwigshafen (DE)**

(56) Entgegenhaltungen:
**EP-A- 0 568 337**
**DE-A- 2 515 033**
**FR-A- 1 440 125**
**DE-A- 2 312 281**
**DE-A- 3 607 883**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 811 003 B1

## Beschreibung

Die vorliegende Erfindung betrifft ein verbessertes Verfahren zur Herstellung von Epoxiden, insbesondere Alkylenoxiden, aus entsprechenden Ausgangsverbindungen, insbesondere den entsprechenden Olefinen, mittels aromatischer Peroxycarbonsäuren.

Die Epoxidierung von Olefinen mit Peroxycarbonsäuren, insbesondere mit m-Chlorperoxybenzoesäure, ist eine für die Synthese von Epoxiden gut etablierte Labormethode.

In der chemischen Fachliteratur ist diese Methode ausführlich beschrieben, beispielsweise durch Y. Sawaki in S. Patai (Herausgeber), Chem. Hydroxyl, Ether Peroxide Groups, S. 590-593 (1993) (1).

Für die Herstellung von Epoxiden im größeren Maßstab ist diese Methode jedoch weniger geeignet, da die Peroxycarbonsäure in stöchiometrischen Mengen eingesetzt und die anfallende Carbonsäure mit großem Aufwand durch Reaktion mit Wasserstoffperoxid regeneriert werden muß.

Der vorliegenden Erfindung lag deshalb die Aufgabe zugrunde, ein Verfahren zur Herstellung von Alkylenoxiden durch Epoxidierung von Olefinen mit aromatischen Peroxycarbonsäuren bereitzustellen, das eine einfach und sicher durchführbare und wirtschaftliche Rückführung der anfallenden Carbonsäure in Peroxycarbonsäure ohne Verwendung von Wasserstoffperoxid ermöglicht.

Demgemäß wurde ein Verfahren zur Herstellung von Epoxiden aus entsprechenden Ausgangsverbindungen mittels aromatischer Peroxycarbonsäuren gefunden, welches dadurch gekennzeichnet ist, daß man nach erfolgter Epoxidation (Schritt A) die entstandenen aromatischen Carbonsäuren von den Epoxiden abtrennt, in Schritt B katalytisch zu den entsprechenden aromatischen Aldehyden hydriert und in Schritt C diese Aldehyde mit Sauerstoff oder einem sauerstoffhaltigen Gasgemisch wieder zu den aromatischen Peroxycarbonsäuren oxidiert, welche erneut zur Herstellung von Epoxiden eingesetzt werden.

Mit diesem Verfahren gemäß Schritt A kann im Prinzip jedes beliebige Olefin epoxidiert werden. Bevorzugt werden Olefine, die direkt an der Doppelbindung nicht mehr als einen elektronenziehenden Substituenten tragen. Besonders bevorzugt sind Olefine ohne elektronenziehende Substituenten an der Doppelbindung. Beispiele für gut einsetzbare Olefine sind lineare oder verzweigte $C_2$- bis $C_{40}$-Olefine, insbesondere $C_3$- bis $C_{24}$-Olefine, oder cyclische Olefine, wie Ethylen, Propen, 1-Buten, 2-Buten, Isobuten, 1-Penten, 2-Penten, 1-Hexen, 1-Hepten, 1-Octen, 2,4,4-Tri-methyl-1-penten, 2,4,4-Trimethyl-2-penten, 1-Nonen, 1-Decen, 1-Dodecen, 1-Tetradecen, 1-Hexadecen, 1-Octadecen, $C_{20}$-Olefin, $C_{22}$-Olefin, $C_{24}$-Olefin, $C_{28}$-Olefin oder $C_{30}$-Olefin, Cyclopropen, Cyclobuten, Cyclopenten, Cyclohexen, Cycloocten, Vinylalkylether wie Vinylmethylether, Vinylethylether oder Vinylbutylether, Allylchlorid, Allylalkohol, Vinylacetat, Vinylpropionat, Styrol sowie Verbindungen mit mehreren olefinischen Doppelbindungen wie 1,3-Butadien, Isopren, Cyclopentadien oder Cyclooctadien. Es können auch Olefinmischungen verwendet werden.

Besonders gut eignet sich das erfindungsgemäße Verfahren für die Epoxidierung von Propen zu Propylenoxid.

Als aromatische Peroxycarbonsäuren eignen sich vor allem Verbindungen der allgemeinen Formel I

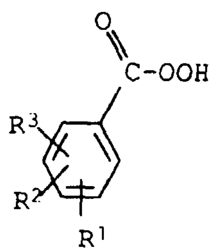

(I)

in der die Substituenten $R^1$ bis $R^3$ unabhängig voneinander für Wasserstoff, $C_1$- bis $C_6$-Alkyl, $C_3$- bis $C_8$-Cycloalkyl, $C_6$- bis $C_{14}$-Aryl, $C_7$- bis $C_{12}$-Phenylalkyl, Halogen, $C_1$- bis $C_6$-Alkoxy, $C_3$-bis $C_8$-Cycloalkoxy $C_6$- bis $C_{14}$-Aryloxy oder $C_7$- bis $C_{12}$-Phenylalkoxy stehen und einer der Substituenten $R^1$ bis $R^3$ auch eine weitere Peroxycarboxylgruppe oder eine Carboxylgruppe bedeuten kann.

Insbesondere haben die Substituenten $R^1$ bis $R^3$ unabhängig voneinander folgende Bedeutung:

- Wasserstoff;

- $C_1$- bis $C_6$-Alkyl, vorzugsweise $C_1$- bis $C_4$-Alkyl wie Methyl, Ethyl, n-Propyl, iso-Propyl, n-Butyl, iso-Butyl, sec.-Butyl, tert.-Butyl, n-Pentyl oder n-Hexyl, insbesondere Methyl oder tert.-Butyl;

- $C_3$- bis $C_8$-Cycloalkyl wie Cyclopropyl, Cyclobutyl, Cyclopentyl, Cyclohexyl, Cycloheptyl oder Cyclooctyl, insbesondere Cyclopentyl oder Cyclohexyl oder substituiertes $C_3$- bis $C_8$-Cycloalkyl, insbesondere 1-Methylcyclopentyl oder 1-Methylcyclohexyl;

- $C_6$- bis $C_{14}$-Aryl wie Phenyl, 1-Naphthyl, 2-Naphthyl, 1-Anthryl, 2-Anthryl oder 9-Anthryl, insbesondere Phenyl;

- $C_7$- bis $C_{12}$-Phenylalkyl wie l-Methyl-1-Phenylethyl, Benzyl, 1-Phenylethyl, 2-Phenylethyl, 1-Phenylpropyl, 2-Phenylpropyl, 3-Phenylpropyl, 1-Phenylbutyl, 2-Phenylbutyl, 3-Phenylbutyl oder 4-Phenylbutyl, insbesondere 1-Methyl-1-phenylethyl;

- Halogen wie Fluor, Chlor oder Brom;

- $C_1$- bis $C_6$-Alkoxy, $C_3$- bis $C_8$-Cycloalkoxy, $C_6$- bis $C_{14}$-Aryloxy oder $C_7$- bis $C_{12}$-Phenylalkoxy, wobei die am Sauerstoffatom stehenden Reste die oben aufgezählten Bedeutungen von $R^1$ bis $R^3$ (mit Ausnahme von Wasserstoff) haben;

- für einen der Substituenten $R^1$ bis $R^3$ Peroxycarboxyl oder Carboxyl.

Weiterhin werden solche aromatischen Peroxycarbonsäuren I bevorzugt, welche ein, zwei oder drei Methylgruppen als Substituenten $R^1$ bis $R^3$ aufweisen.

Beispiele für einsetzbare aromatische Peroxycarbonsäuren sind insbesondere Peroxybenzoesäure, 2-Methylperoxybenzoesäure (o-Peroxytoluylsäure), 3-Methylperoxybenzoesäure (m-Peroxytoluylsäure), 4-Methylperoxybenzoesäure (p-Peroxytoluylsäure), 2,4- und 3,5-Dimethylperoxybenzoesäure, 2,4,6-Trimethylperoxybenzoesäure, 4-tert.-Butylperoxybenzoesäure, 2-Methyl-4-tert.-butylperoxybenzoesäure, 2,6-Dimethyl-4-tert.-butylperoxybenzoesäure, 2-, 3-oder 4-Ethylperoxybenzoesäure, 4-(1-Methylcyclohexyl)peroxybenzoesäure, 4-(1-Methylcyclopentyl)peroxybenzoesäure, 4-Phenylperoxybenzoesäure, 3-Chlorperoxybenzoesäure, 4-Methoxy- oder 4-Ethoxyperoxybenzoesäure, 4-Methoxy- oder 4-Ethoxy-2,6-dimethyl-peroxybenzoesäure, Bisperoxyphthalsäure, Monoperoxyphthalsäure, Bisperoxyterephthalsäure und Monoperoxyterephthalsäure. Es können auch Gemische der genannten aromatischen Peroxycarbonsäuren eingesetzt werden. Besonders bevorzugt ist o-Peroxytoluylsäure.

Schritt A des erfindungsgemäßen Verfahrens ist hinsichtlich Epoxidierung von Olefinen in der Literatur beschrieben. Die Epoxidierung wird in der Regel folgendermaßen durchgeführt:

Die aromatische Peroxycarbonsäure, gelöst in einem geeigneten Lösungsmittel, wird mit einem Olefin zur Reaktion gebracht. Das molare Verhältnis Olefin zu Peroxycarbonsäure liegt bei 0,8:1 bis 100:1, insbesondere 1:1 bis 20:1, vor allem 1,5:1 bis 5:1.

Die eingesetzte Peroxycarbonsäurelösung kann in einem Lösungsmittel aufgelöste isolierte Peroxycarbonsäure sein. Bevorzugt wird jedoch direkt die Lösung eingesetzt, die im Oxidationsschritt C hergestellt wurde (gegebenenfalls nach einem Reinigungsschritt, in dem die Peroxycarbonsäure in Lösung bleibt).

Als organische Lösungsmittel für die Peroxycarbonsäuren bei der Epoxidierung können Ketone (z.B. Aceton, Butanon oder tert.-Butylmethylketon), Ester (z.B. Methyl- und Ethylacetat oder Methylbenzoat), Nitroverbindungen (z.B. Nitromethan oder Nitrobenzol), halogenierte Kohlenwasserstoffe (z.B. Di- und Trichlormethan, 1,1,1-Trichlorethan oder Chlorbenzol), Carbonate (z.B. Dimethylcarbonat), Harnstoffderivate (z.B. Tetramethylharnstoff), anorganische Ester oder Amide (z.B. Trimethylphosphat oder Hexamethylphosphorsäuretriamid), Kohlenwasserstoffe (z.B. Hexan oder Heptan) oder Alkylaromaten (z.B. Benzol, Toluol oder Xylol) verwendet werden. Besonders bevorzugt wird jedoch die Verwendung desselben Lösungsmittels wie bei der Oxidation in Schritt C. Besonders bevorzugte Lösungsmittel für beide Schritt sind Aceton, Methylacetat und Ethylacetat.

Die Epoxidierung kann bei -20 bis 100°C durchgeführt werden, je nach Lösungsmittel und Olefin. Bei Verwendung von Aceton als Lösungsmittel und terminalen Olefinen (wie z.B. 1-Octen oder Propen) als Substrat werden Temperaturen von 25 bis 80°C bevorzugt. Besonders bevorzugt werden Temperaturen von 45 bis 65°C.

Überraschenderweise reagiert bei der relativ hohen Temperatur von 45°C oder darüber das Olefin viel schneller zum Epoxid als gegebenenfalls noch vorhandener aromatischer Aldehyd aus Stufe B zur Carbonsäure ab.

Die Abtrennung der in Schritt A aus den aromatischen Peroxycarbonsäuren I entstandenen aromatischen Carbonsäuren von den Oxidationsprodukten, insbesondere den Alkylenoxiden, geschieht nach üblichen Methoden, beispielsweise durch Filtration, Extraktion oder Destillation.

Die katalytische Hydrierung der aromatischen Carbonsäuren in Schritt B erfolgt vorzugsweise in der Gasphase mit Wasserstoff in Gegenwart eines Lanthanid/Zirkonoxid-Katalysators. Derartige Katalysatoren sind als Hydrierungskatalysatoren für die Überführung von aromatischen Carbonsäuren in die entsprechenden Aldehyde aus der deutschen Patentanmeldung P 44 28 994.4 (2) bekannt.

Schritt B des erfindungsgemäßen Verfahrens wird zweckmäßigerweise wie folgt durchgeführt:

Die Hydrierung der aromatischen Carbonsäuren mit Wasserstoff in Gegenwart eines Katalysators, dessen katalytisch aktive Masse 60 bis 99,9, insbesondere 80 bis 99,9 Gew.-% Zirkonoxid ($ZrO_2$) und 0,1 bis 40, insbesondere 0,1 bis 20 Gew.-% eines oder mehrere Elemente der Lanthaniden enthält, wird in der Regel bei Temperaturen von 200 bis 450°C, vorzugsweise 250 bis 400°C, insbesondere 300 bis 380°C, und Drücken von 0,1 bis 20 bar, vorzugsweise 0,7 bis 5 bar, insbesondere Atmosphärendruck (Normaldruck), durchgeführt. Die erforderliche Temperatur und der erforderliche Druck sind abhängig von der Katalysatoraktivität und der thermischen Stabilität von Edukt und Produkt.

Als Katalysatoren eignen sich Trägerkatalysatoren, vorzugsweise Vollkatalysatoren von Zirkonoxid in kubischer, tetragonaler oder monokliner Phase, vorzugsweise in monokliner Phase, die mit einem oder mehreren Elementen aus der Lanthaniden-Reihe dotiert sind. Die katalytisch aktive Masse enthält vorzugsweise 90 bis 99,9 Gew.-%, insbesondere 92 bis 99 Gew.-% Zirkonoxid und 0,1 bis 10 Gew.-%, insbesondere 1 bis 8 Gew.-%, eines oder mehrere Elemente der Lanthaniden, insbesondere Lanthan, Cer, Praseodym, Neodym, Samarium, Europium oder deren Gemische, vor allem Lanthan als Lanthan-(III)-oxid. Die Dotierung erfolgt in der Regel durch Tränken des Zirkonoxids mit Salzlösungen (wäßrig oder alkoholisch) der Lanthaniden.

Der Katalysator kann zusätzlich weitere Dotierungen (z.B. Chrom, Eisen, Yttrium, Hafnium, Mangan) in Mengen von 0,001 bis 10 Gew.-% enthalten. Bevorzugt sind jedoch Katalysatoren ohne solche weiteren Zusätze.

Die BET-Oberfläche des Zirkonoxids kann in weiten Grenzen schwanken, beträgt in der Regel aber 5 bis 150 $m^2$/g, vorzugsweise 20 bis 150 $m^2$/g, insbesondere 40 bis 120 $m^2$/g.

Derartige Katalysatoren werden in bekannter Weise z.B. durch Tränken vorgeformter Träger wie Pellets, Kugeln oder Stränge, Trocknen und Calcinieren hergestellt.

Die bevorzugt verwendeten Trägerkatalysatoren zeigen über einen längeren Zeitraum hohe Aktivität. Desaktivierte Katalysatoren lassen sich durch Behandlung mit molekularen Sauerstoff enthaltenden Gasen, z.B. Luft, bei Temperaturen von 350 bis 500°C regenerieren.

Im allgemeinen hält man eine Katalysatorbelastung von 0,01 bis 10, vorzugsweise 0,01 bis 3 kg aromatischer Carbonsäure je kg Katalysator und Stunde ein.

Die Wasserstoffkonzentration im Eingangsgas richtet sich nach der Carbonsäurekonzentration. Das Molverhältnis von Wasserstoff zu aromatischer Carbonsäure beträgt in der Regel 2:1 bis 100:1, bevorzugt 10:1 bis 70:1. Als Wasserstoffquelle kann auch Ameisensäure eingesetzt werden.

Vorteilhaft kann auch der Zusatz eines inerten Verdünnungsmittels sein. In der Regel werden Stickstoff, Wasser oder gasförmige, unter den Reaktionsbedingungen inerte Verbindungen wie Kohlenwasserstoffe, Aromaten oder Ether verwendet.

Die Umsetzung kann in der Gasphase entweder kontinuierlich als Festbettreaktion mit fest angeordnetem Katalysator, beispielsweise in Sumpf- oder Rieselfahrweise, oder als Wirbelbettreaktion mit in auf- und abwirbelnder Bewegung befindlichem Katalysator durchgeführt werden. Bevorzugt ist das Arbeiten im Festbett.

Zur Steigerung der Selektivität können bei der Hydrierung gebildete Nebenprodukte, z.B. Alkohole, in die Synthese zurückgeführt werden.

Das den aromatischen Aldehyd enthaltende Gemisch aus Schritt B wird, gegebenenfalls nach einem Reinigungsschritt, in Schritt C zweckmäßigerweise in einem geeigneten Lösungsmittel aufgenommen und in der Flüssigphase mit Sauerstoff oder einem sauerstoffhaltigen Gasgemisch zur entsprechenden aromatischen Percarbonsäure oxidiert. Dabei arbeitet man vorzugsweise bei Temperaturen von -10°C bis 100°C und Sauerstoff-Partialdrücken von 0,001 bis 100 bar.

Aus der DE-A 25 15 033 (3) ist bekannt, daß man p-Toluylaldehyd in Acetonlösung mit Luft bei 28°C und 30 bar ohne Katalysator mit ca. 80 % Ausbeute zu p-Peroxytoluylsäure oxidieren kann. Solch hohe Ausbeuten werden aber nur erreicht, wenn hochreiner p-Toluylaldehyd und wasserfreies Aceton eingesetzt werden.

Schritt C des erfindungsgemäßen Verfahrens wird normalerweise folgendermaßen durchgeführt:

Die Konzentration des aromatischen Aldehyds im Lösungsmittel kann 1 bis 75 Gew.-% betragen. Bevorzugt werden dabei Konzentrationen von 5 bis 35 Gew.-%, insbesondere von 8 bis 20 Gew.-%.

Sauerstoff oder das sauerstoffenthaltende Gasgemisch kann entweder gasförmig oder als Lösung, gegebenenfalls auch unter Druck, mit dem aromatischen Aldehyd zur Reaktion gebracht werden. Der Sauerstoffpartialdruck beträgt vorzugsweise 0,01 bis 30 bar, insbesondere 0,05 bis 5 bar.

Die Oxidation kann ein- oder zweiphasig durchgeführt werden. Für die einphasige Fahrweise eignen sich Reaktoren, in denen man eine Lösung des aromatischen Aldehyds mit einer Lösung von Sauerstoff, gegebenenfalls unter Druck, zur Reaktion bringen kann, z.B. Rohrreaktoren oder geflutete Rührkessel. Für die zweiphasige Fahrweise eignen sich Reaktoren, die eine gute Gas/Flüssig-Durchmischung gewährleisten, wie Blasensäulen (mit oder ohne Trennbleche bzw. Füllkörper), Rührkessel (gegebenenfalls mit Begasungsrührer und gegebenenfalls als Kaskade angeordnet) oder Rieselreaktoren.

Die Reaktionstemperatur beträgt vorzugsweise 0 bis 600C, insbesondere 15 bis 40°C.

Die Reaktionszeit wird so gewählt, daß ein Aldehydumsatz zwischen 40 und 100 % erreicht wird. Bevorzugt sind Reaktionszeiten, mit denen ein Aldehydumsatz zwischen 60 und 99 % erreicht wird. Besonders bevorzugt sind Reaktionszeiten, mit denen ein Aldehydumsatz zwischen 75 und 95 % erreicht wird.

Bei der Oxidation kann auch ein Stabilisator für die gebildete Peroxycarbonsäure, z.B. 8-Hydroxychinolin, Dipicolinsäure oder 2,6-Dihydroxymethylpyridin, zugesetzt werden.

Als organische Lösungsmittel für Schritt C können Ketone (z.B. Aceton, Butanon oder tert.-Butyl-methylketon), Ester (z.B. Methyl- und Ethylacetat oder Methylbenzoat), Nitroverbindungen (z.B. Nitromethan oder Nitrobenzol), halogenierte Kohlenwasserstoffe (z.B. Di- und Trichlormethan, 1,1,1-Trichlorethan oder Chlorbenzol), Carbonate (z.B. Dimethylcarbonat), Harnstoffderivate (z.B. Tetramethylharnstoff), anorganische Ester oder Amide (z.B. Trimethylphosphat oder Hexamethylphosphorsäuretriamid) oder Alkylaromaten (z.B. Benzol, Toluol oder Xylol) verwendet werden. Bevorzugt sind Ketone, insbesondere Aceton und tert.-Butyl-methylketon, und Ester, insbesondere Methyl- und Ethylacetat und Methylbenzoat.

Die aromatische Percarbonsäure kann entweder isoliert werden (z.B. durch Fällung), aber auch ohne Isolierung (d.h. in Lösung) direkt wieder in Schritt A eingesetzt werden.

Überraschend ist, daß sich o-Tolylaldehyd schneller und selektiver oxidieren läßt als die isomeren m- und p-Tolylaldehyde.

Das erfindungsgemäße Verfahren hat den Vorteil, daß die aromatische Peroxycarbonsäure nach der Oxidation bzw. Epoxidierung ohne Einsatz von Wasserstoffperoxid regeneriert wird. Die aromatische Peroxycarbonsäure fungiert nur als Sauerstoffüberträger und wird praktisch nicht verbraucht. Die Stöchiometrie für das Gesamtverfahren lautet im Falle der Epoxidierung:

$$\text{Olefin} + O_2 + H_2 \rightarrow \text{Alkylenoxid} + H_2O.$$

Ein Reaktionsschema für die Epoxidierung unter Verwendung der aromatischen Peroxycarbonsäuren I sieht folgendermaßen aus:

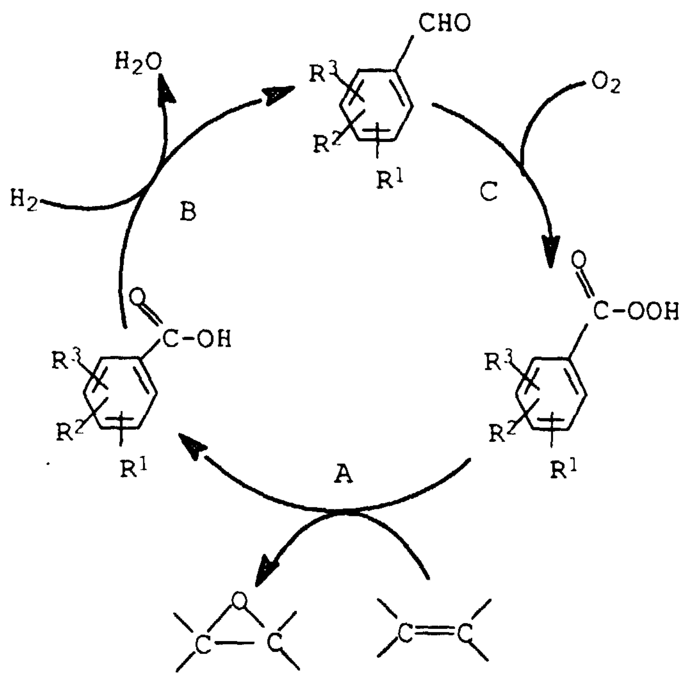

(I)

Beispiele

Beispiel 1

Epoxidierung von 1-Octen mit p-Peroxytoluylsäure in Aceton

50 g einer 8,3 gew.-%igen Lösung von p-Peroxytoluylsäure in Aceton wurden mit 4,6 g (1,5 Äquivalenten) 1-Octen versetzt und bei 40°C gerührt. Nach 5 Stunden war die Peroxysäure zu ca. 90 % umgesetzt. Die Octenoxid-Selektivität betrug ca. 80 %, bezogen auf die Peroxysäure, und >95 %, bezogen auf 1-Octen. Die Reaktionstemperatur konnte ohne wesentliche Verminderung der Selektivität erhöht werden. Bei 60°C Reaktionstemperatur lag der Peroxysäure-Umsatz nach 2 Stunden schon bei ca. 90 %. Die Octenoxid-Selektivität blieb gegenüber dem Versuch bei 40°C unverändert.

Beispiel 2

Epoxidierung von Propen mit p-Peroxytoluylsäure in Aceton

35 g einer 8,4 gew.-%igen Lösung von p-Peroxytoluylsäure in Aceton wurden in einem 50 ml Glasautoklav vorgelegt, 2,4 g Propen (3 Äquivalente) aufgepreßt und es wurde 4,5 Stunden bei 600C gerührt. Der Peroxysäure-Umsatz betrug 94 %. Die Propylenoxid-Selektivität, bezogen auf die Peroxysäure, war >95 %.

Beispiel 3

Epoxidierung von 1-Octen mit o-Peroxytoluylsäure in Aceton

100 g einer 11,3 gew.-%igen Lösung von o-Peroxytoluylsäure in Aceton wurden mit 16,8 g 1-Octen (2 Äquivalenten) versetzt und bei 60°C gerührt. Nach 1 Stunde war der Peroxysäure-Umsatz 92 %. Die Octenoxid-Selektivität betrug 97 %, bezogen auf o-Peroxytoluylsäure.

Beispiel 4

Herstellung des Katalysators für die Hydrierung in Schritt B

Monoklines $ZrO_2$ (BET-Oberfläche: 40 bis 85 $m^2/g$) in Form von Tabletten (Katalysator A und E) oder Strängen (Katalysator B, C und D) wurde mit einer wäßrigen Lösung des Lanthanid-Element-Nitrats (bzw. der Lanthanid-Element-Nitrate) unter guter Durchmischung getränkt und 2 Stunden bei Raumtemperatur gehalten. Anschließend wurde der Katalysator 15 Stunden bei 120°C getrocknet und anschließend 2 bis 4 Stunden bei 400 bis 500°C getempert.

Die so hergestellten Katalysatoren hatten folgenden Lanthanid-Gehalt:

Katalysator A (Oberfläche 67 $m^2/g$): 3 Gew.-% Lanthan;
Katalysator B (Oberfläche 46 $m^2/g$): 3 Gew.-% Praseodym;
Katalysator C (Oberfläche 46 $m^2/g$): 3 Gew.-% Cer;
Katalysator D (Oberfläche 46 $m^2/g$): 3 Gew.-% Lanthanide (Verteilung: 48,2 Gew.-% $CeO_2$, 26,4 Gew.-% $La_2O_3$, 5,7 Gew.-% $Pr_2O_3$ und 19,7 Gew.-% $Nd_2O_3$);
Katalysator E (Oberfläche 53 $m^2/g$): 3 Gew.-% Lanthan.

Beispiele 5a bis 5i

Hydrierung von 4-substituierten aromatischen Carbonsäuren

Pro Stunde wurden 4 bis 8 g aromatische Carbonsäure, gegebenenfalls gelöst in Tetrahydrofuran (THF), in einen Verdampfer (<300°C) und von dort mit 100 l/h Wasserstoff über 100 g Katalysator in Rieselfahrweise geleitet. Der gasförmige Reaktionsaustrag wurde in Kühlfallen kondensiert und gaschromatographisch analysiert. Die eingesetzten Carbonsäuren und die Ergebnisse sind in der Tabelle 1 zusammengefaßt.

Tabelle 1

| Bsp. Nr. | Katalysator | Carbonsäure R[1)] | Konz. der Carbonsäure [Gew.-%][2)] | Reaktortemp. [°C] | Ausbeute an Aldehyd [%] | Umsatz [%] | Selektivität [%] |
|---|---|---|---|---|---|---|---|
| 5a | A | H | 100 | 340 | 98 | 100 | 98 |
| 5b | A | H | 20 | 350 | 98 | 100 | 98 |
| 5c | A | Methyl | 100 | 340 | 96 | 99 | 97 |
| 5d | A | t-Butyl | 100 | 340 | 90 | 94 | 96 |
| 5e | A | t-Butyl | 20 | 340 | 93 | 97 | 96 |
| 5f | A | Methyl | 10 | 350 | 77 | 99 | 78 |
| 5g | B | H | 100 | 360 | 95 | 100 | 95 |
| 5h | C | H | 100 | 360 | 96 | 100 | 96 |
| 5i | D | H | 100 | 360 | 97 | 99 | 98 |

1) Substituent an der 4-Position der Carbonsäure 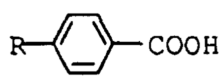

2) im Lösungsmittel (THF), bei 100 Gew.-% reine Carbonsäure ohne Lösungsmittel

Beispiel 6

Hydrierung von 3-Methylbenzoesäure

Pro Stunde wurden 8 g 3-Methylbenzoesäure (als Schmelze) mit 100 l/h Wasserstoff verdampft und bei 360°C über 100 g Katalysator E in Rieselfahrweise geleitet. Der gasförmige Reaktionsaustrag wurde in Kühlfallen kondensiert und gaschromatographisch analysiert. Die 3-Methylbenzaldehyd-Ausbeute betrug 92 % (Umsatz 99 %).

Beispiel 7

Hydrierung von 2-Methylbenzoesäure

Pro Stunde wurden 8 g 2-Methylbenzoesäure (als Schmelze) mit 200 l/h Wasserstoff verdampft und bei 350°C über 100 g Katalysator E in Rieselfahrweise geleitet. Der gasförmige Reaktionsaustrag wurde in Kühlfallen kondensiert und gaschromatographisch analysiert. Die 2-Methylbenzaldehyd-Ausbeute betrug 93 % (Umsatz 99 %).

Beispiele 8a bis 8e

Oxidation von aromatischen Aldehyden mit Luft zu Peroxycarbonsäuren in Aceton

Eine Lösung von aromatischem Aldehyd (10 gew.-%ig in Aceton) wurde in einem Vierhalskolben mit Gaseinleitungsrohr, Hochgeschwindigkeits-Hoesch-Rührer, Thermometer und Rückflußkühler bei 30"C mit Luft oxidiert. Die Peroxysäurekonzentration wurde iodome-trisch bestimmt. Andere Komponenten können durch Gaschromatographie bestimmt werden (nach Reduktion der Peroxysäure mit Tributylphosphit). Die eingesetzten Aldehyde und die Ergebnisse sind in der Tabelle 2 zusammengefaßt.

Tabelle 2

| Bsp. Nr. | Aldehyd | Reaktionszeit [h] | Umsatz an Aldehyd [%] | Peroxysäure-Selektivität [%] |
|---|---|---|---|---|
| 8a | Benzaldehyd | 2 | 34 | 77 |
| 8b | p-Toluylaldehyd | 7 | 84 | 83 |
| 8c | m-Toluylaldehyd | 6 | 90 | 82 |
| 8d | o-Toluylaldehyd | 4 | 80 | 93 |
| 8e | p-Methoxybenzaldehyd | 1 | 37 | 72 |

Beispiel 9

Oxidation von p-Toluylaldehyd in Methylacetat

p-Toluylaldehyd wurde wie im Beispiel 8b oxidiert, aber mit Methylacetat statt Aceton als Lösungsmittel. Nach 7 Stunden Reaktionszeit war der Aldehyd zu 62 % umgesetzt. Die Selektivität zu p-Toluylperoxysäure betrug 69 %.

Beispiel 10

Oxidation von o-Toluylaldehyd mit Sauerstoff unter Druck

Eine 10 gew.-%ige Lösung von o-Toluylaldehyd in Aceton wurde in einem magnetisch gerührten 10 ml Glasautoklav mit reinem Sauerstoff bei 5 bar und 30°C oxidiert. Nach 1,5 Stunden waren ca. 80 % des Aldehyds umgesetzt. Die o-Toluylperoxysäure wurde mit einer Selektivität von >90 % gebildet. Der Rest bestand hauptsächlich aus o-Toluylsäure. Nebenprodukte wie Phthalid, Toluol, o-Cresol und o-Cresolformiat bildeten sich nur zu ca. 0,2 %.

Die Oxidation konnte auch in konzentrierteren Lösungen durchgeführt werden. Die Oxidation einer 20 gew.-%igen Lösung von o-Toluylaldehyd (30°C, 5 bar Sauerstoff, 3 Stunden Reaktionszeit) lieferte die entsprechende Peroxysäure mit einer Selektivität von ca. 93 % (Aldehydumsatz: 90 %).

**Patentansprüche**

1. Verfahren zur Herstellung von Alkylenoxiden aus den entsprechenden Olefinen mittels aromatischer Peroxycarbonsäuren, dadurch gekennzeichnet, daß man nach erfolgter Epoxidierung der Olefine (Schritt A) die entstandenen aromatischen Carbonsäuren von den Alkylenoxiden abtrennt, in Schritt B katalytisch zu den entsprechenden aromatischen Aldehyden hydriert und in Schritt C diese Aldehyde mit Sauerstoff oder einem sauerstoffhaltigen Gasgemisch wieder zu den aromatischen Peroxycarbonsäuren oxidiert, welche erneut zur Epoxidierung von Olefinen eingesetzt werden.

2. Verfahren nach Anspruch 1, dadurch gekennzeichnet, daß man hiermit Propen zu Propylenoxid epoxidiert.

3. Verfahren nach den Ansprüchen 1 oder 2, dadurch gekennzeichnet, daß man als aromatische Peroxycarbonsäuren Verbindungen der allgemeinen Formel I

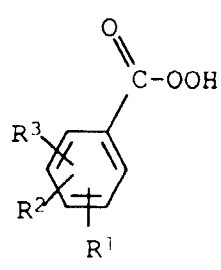

(I)

in der die Substituenten $R^1$ bis $R^3$ unabhängig voneinander für Wasserstoff, $C_1$- bis $C_6$-Alkyl, $C_3$- bis $C_8$-Cycloalkyl, $C_6$- bis $C_{14}$-Aryl, $C_7$- bis $C_{12}$-Phenylalkyl, Halogen, $C_1$- bis $C_6$-Alkoxy, $C_3$- bis $C_8$-Cycloalkoxy, $C_6$- bis $C_{14}$-Aryloxy oder $C_7$- bis $C_{12}$-Phenylalkoxy stehen und einer der Substituenten $R^1$ bis $R^3$ auch eine weitere Peroxycarboxylgruppe oder eine Carboxylgruppe bedeuten kann, einsetzt.

**4.** Verfahren nach den Ansprüchen 1 bis 3, dadurch gekennzeichnet, daß man die katalytische Hydrierung der aromatischen Carbonsäuren in Schritt B in der Gasphase mit Wasserstoff in Gegenwart eines Lanthanid/Zirkonoxid-Katalysators durchführt.

**5.** Verfahren nach den Ansprüchen 1 bis 3, dadurch gekennzeichnet, daß man die Oxidation der aromatischen Aldehyde in Schritt C in der Flüssigphase in einem geeigneten Lösungsmittel bei Temperaturen von -10°C bis 100°C und Sauerstoff-Partialdrücken von 0,001 bis 100 bar durchführt.

**6.** Verfahren gemäß den Ansprüchen 1 bis 3, dadurch gekennzeichnet, daß man die Epoxidation in einem Lösungsmittel vornimmt.

**7.** Verfahren gemäß Anspruch 1, dadurch gekennzeichnet, daß man Epoxidation und Oxidation der Aldehyde im gleichen Lösungsmittel durchführt.

**8.** Verfahren gemäß Anspruch 7, dadurch gekennzeichnet, daß man als Lösungsmittel Aceton, Methylacetat oder Ethylacetat verwendet.

**9.** Verfahren gemäß Anspruch 3, dadurch gekennzeichnet, daß man als Peroxycarbonsäure o-Peroxytoluylsäure verwendet.

## Claims

**1.** A process for preparing an alkylene oxide from the corresponding olefin by means of an aromatic peroxycarboxylic acid, which comprises a step A of epoxidizing the olefin and removing the resulting aromatic carboxylic acid from the alkylene oxide, a step B of catalytically hydrogenating the removed aromatic carboxylic acid to the corresponding aromatic aldehyde, and a step C of oxidizing this aldehyde with oxygen or an oxygen-containing gas mixture back to the aromatic peroxycarboxylic acid for re-use for epoxidizing an olefin.

**2.** A process as claimed in claim 1 wherein propene is epoxidized to propylene oxide.

**3.** A process as claimed in claim 1 or 2 wherein the aromatic peroxycarboxylic acid used is a compound of the general formula I

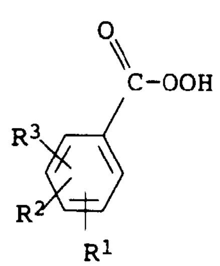

(I)

where $R^1$ to $R^3$ are independently of one another hydrogen, $C_1$-$C_6$-alkyl, $C_3$-$C_8$-cycloalkyl, $C_6$-$C_{14}$-aryl, $C_7$-$C_{12}$-phenylalkyl, halogen, $C_1$-$C_6$-alkoxy, $C_3$-$C_8$-cycloalkoxy, $C_6$-$C_{14}$-aryloxy or $C_7$-$C_{12}$-phenylalkoxy and one of $R^1$ to $R^3$ can also be a further peroxycarboxyl group or a carboxyl group.

**4.** A process as claimed in any of claims 1 to 3 wherein the step B catalytic hydrogenation of the aromatic carboxylic acid is carried out with hydrogen in the gas phase in the presence of a lanthanide/zirconia catalyst.

**5.** A process as claimed in any of claims 1 to 3 wherein the step C oxidation of the aromatic aldehyde is carried out

in the liquid phase in a suitable solvent at temperatures from -10°C to 100°C and oxygen partial pressures from 0.001 to 100 bar.

6. A process as claimed in any of claims 1 to 3 wherein the epoxidation is carried out in a solvent.

7. A process as claimed in claim 1 wherein the epoxidation and the oxidation of the aldehyde are carried out in the same solvent.

8. A process as claimed in claim 7 wherein the solvent used is acetone, methyl acetate or ethyl acetate.

9. A process as claimed in claim 3 wherein the peroxycarboxylic acid used is o-peroxytoluic acid.

**Revendications**

1. Procédé de préparation d'oxydes d'alkylène à partir des oléfines correspondantes, à l'aide d'acides peroxycarboxyliques aromatiques, caractérisé en ce que, après qu'a eu lieu une époxydation des oléfines (étape A), on sépare des oxydes d'alkylène les acides carboxyliques aromatiques obtenus, on les soumet dans l'étape B à une hydrogénation catalytique pour donner les aldéhydes aromatiques correspondants, et, dans l'étape C, on oxyde de nouveau ces aldéhydes, avec de l'oxygène ou un mélange gazeux contenant de l'oxygène, pour obtenir les acides peroxycarboxyliques aromatiques, qui sont de nouveau utilisés pour l'époxydation d'oléfines.

2. Procédé selon la revendication 1, caractérisé en ce qu'on époxyde grâce à lui du propène en oxyde de propylène.

3. Procédé selon les revendications 1 ou 2, caractérisé en ce qu'on utilise en tant qu'acides peroxycarboxyliques aromatiques des composés de formule générale I

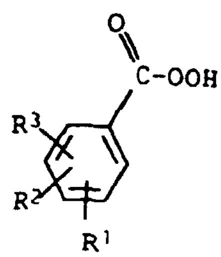

(I)

dans laquelle les substituants $R^1$ à $R^3$ représentent, indépendamment les uns des autres, des atomes d'hydrogène, des groupes alkyle en $C_1$ à $C_6$, cycloalkyle en $C_3$ à $C_8$, aryle en $C_6$ à $C_{14}$, phénylalkyle en $C_7$ à $C_{12}$, halogéno, alcoxy en $C_1$ à $C_6$, cycloalcoxy en $C_3$ à $C_8$, aryloxy en $C_6$ à $C_{14}$ ou phénylalcoxy en $C_7$ à $C_{12}$, et l'un des substituants $R^1$ à $R^3$ peut aussi être un autre groupe peroxycarboxyle ou un groupe carboxyle.

4. Procédé selon l'une quelconque des revendications 1 à 3, caractérisé en ce que l'hydrogénation catalytique des acides carboxyliques aromatiques dans l'étape B est effectuée en phase gazeuse avec de l'hydrogène en présence d'un catalyseur à base de lanthanide/oxyde de zirconium.

5. Procédé selon l'une quelconque des revendications 1 à 3, caractérisé en ce que l'oxydation des aldéhydes aromatiques dans l'étape C est effectuée en phase liquide dans un solvant approprié à des températures de -10 à 100°C et sous des pressions partielles d'oxygène de 0,001 à 100 bar.

6. Procédé selon l'une quelconque des revendications 1 à 3, caractérisé en ce qu'on procède à l'époxydation dans un solvant.

7. Procédé selon la revendication 1, caractérisé en ce qu'on met en oeuvre l'époxydation et l'oxydation des aldéhydes dans le même solvant.

8. Procédé selon la revendication 7, caractérisé en ce qu'on utilise en tant que solvant de l'acétone, de l'acétate de

méthyle ou de l'acétate d'éthyle.

9. Procédé selon la revendication 3, caractérisé en ce qu'on utilise en tant qu'acide peroxycarboxylique l'acide o-peroxytoluylique.